# EUROPEAN PATENT SPECIFICATION

(11) **EP 1 615 604 B1**
(45) Date of publication and mention of the grant of the patent: **12.08.2009**
(21) Application number: 04750224.0
(22) Date of filing: 16.04.2004
(51) Int. Cl.: A61F 9/007

(54) **OPTHALMIC MICROSURGICAL INSTRUMENTS**
MIKROCHIRURGISCHE INSTRUMENTE FÜR DIE OPHTHALMOLOGIE
INSTRUMENTS DE MICROCHIRURGIE OPHTALMIQUE

(30) Priority: 16.04.2003 US 463549 P
(43) Date of publication of application: 18.01.2006
(73) Proprietor: iScience Interventional Corporation, San Francisco, CA 94117 (US)
(72) Inventor: CONSTON, Stanley, R.,, San Carlos, CA 94070 (US); KUPIECKI, David, J.,, San Francisco, CA 94117 (US); MCKENZIE, John, San Carlos, CA 94070 (US); YAMAMOTO, Ronald, K.,, San Francisco, CA 94117 (US)
(74) Representative: Stainthorpe, Vanessa Juliet
(86) International application number: PCT/US2004/011783
(87) International publication number: WO 2004/093761

(56) References cited:
- WO-A-03/043549
- WO-A-03/045290
- US-A- 4 501 274
- US-A- 5 891 084
- US-A- 6 142 990
- US-A1- 2001 053 873

## Description

### Background of Invention:

Glaucoma is a disease condition of the eye in which increased intraocular pressure (IOP) is created by blockage of the drainage mechanism for the aqueous fluid produced in the anterior portion of the eye. Such conditions are usually treated by topical drugs in the form of eye drops, but may result in surgical treatment if drug treatment becomes ineffective or if patient compliance is an issue. Traditional glaucoma surgery such as trabeculectomy, involves a flap dissection of the eye and the removal of a portion of the trabecular meshwork (TM) or the corneo-scleral junction. The aqueous fluid is directed posteriorly under the surgical flap and to a sub-conjunctival lake known as a bleb. Post-surgical complications and bleb management are significant issues with trabeculectomy and similar procedures. Furthermore, the control of the aqueous outflow is achieved through the management of the integrity of the surgical flap rather than controlling the opening into the anterior chamber. Other procedures involving laser energy to create holes in the TM are partially successful, however long term results are limited as compared to trabeculectomy.

Recently developed surgical treatments for glaucoma involve surgically accessing Schlemm's Canal by manner of a surgical flap or flaps and subsequently dilating or expanding the canal to increase aqueous humor drainage into the natural drainage pathway. Current procedures and instruments can only access a short passage of Schlemm's Canal from either side of the surgical site. US 5,486,165 to Stegmann et al. in discloses a microcannula designed for delivery of substances to Schlemm's Canal during such a procedure. EP 0898947A2 to Grieshaber et al. discloses an improvement to the Stegmann apparatus to deliver substances or stents for maintaining the passage of fluid in the canal. Other inventions disclose the use of microcatheters to introduce water-jet type cutting apparatus or bladed mechanisms to the canal for disruption of the TM. However these methods cut the TM network open in a non-controlled manner and do not remove tissue or debris from the operative field.

The treatment of glaucoma usually involves patient specific requirements for the amount of drainage increase desired by the physician. It is therefore of advantage to be able to treat or remove a controlled amount of the TM or associated juxtacanalicular tissues in order to be able to titrate drainage rates and control the disease process on a patient specific basis. Furthermore, it is desired to perform the controlled treatment or removal of tissues from within Schlemm's Canal in order to facilitate the restoration of natural aqueous drainage system without the requirement for blebs and the concomitant complications, and to enable less invasive surgical methods. It is also advantageous to physically stabilize the tissues in order to facilitate control of the amount of tissues being treated or removed.

This invention is directed at ophthalmic microsurgical instruments which may be directly inserted into Schlemm's Canal to allow controlled treatment or removal of adjacent tissues such as the TM or the juxtacanalicular tissues to effect the reduction of intra-ocular pressure. It is a further object of this invention to describe an instrument which allows the directed access to Schlemm's Canal by a flexible microcannula. The instrument is useful in allowing controlled guidance by the surgeon while viewing through a surgical microscope or by non-invasive medical imaging.

### Known prior art:

United States Patent 4,501,274 (Skjaerpe), Microsurgical instrument
United States Patent 5,486,165 (Stegmann), Method and appliance for maintaining the natural intraocular pressure
United States Patent 5,891,084 (Lee), Multiple chamber catheter delivery system
United States Patent 6,142,990 (Burk), Medical apparatus especially for lowering intraocular pressure by acting on the Schlemm's canal and on the tissue of the trabecular meshwork directly in contact with the Schlemm's canal, including a probe containing a photoconductor which is curved or elastically or plastically deformable to a curved configuration and which is connected to a laser, the photoconductor having a surface coating having at least one emission window therein facing the inner curvature of the photoconductor, the emission window permitting a laser beam to issue from the photoconductor at an angle of 90 degrees to the curvature of the photoconductor.
United States Patent 6,221,078 (Bylsma), Surgical implantation apparatus
United States Patent 6,283,940 (Mulholland), Catheter
United States Patent 6,375,642 B1 (Grieshaber et al), Method of and device for improving drainage of aqueous humor within the eye
United States Patent 6,494,857 B1 (Neuhann), Device for improving in a targeted manner and/or permanently ensuring the ability of the aqueous humor to pass through the trabecular meshwork
United States Patent 6,764,439 (Schaaf et al) discloses a device for improving drainage of the aqueous humor within the eye comprising a rigid probe 30 insertable through a slit-like incision into the anterior chamber to create a fluid pathway between the anterior chamber and Schlemm's Canal. This document constitutes the preamble of claim 1.
United States Patent Application 20020013546 (Grieshaber et al), Method and device to improve aqueous humor drainage in an eye
United States Patent Application 20020111608 (Baerveldt et al), Minimally invasive glaucoma surgical instrument and method
United States Patent Application 20020082591 (Haefliger), Device for the treatment of glaucoma
United States Patent Application 2003014092 (Neuhann), Apparatus for the treatment of glaucoma
Patent Number: EP0898947 A2 (Inventor Grieshaber et al), Method and apparatus to improve the outflow of the aqueous humor of an eye
Patent Number: EP1114627 A1 (Inventor Grieshaber et al), Method and apparatus to improve the outflow of the aqueous humor of an eye
Patent Number: W00064389 (Inventor Brown et al), Trabeculotomy device and method for treating glaucoma
Patent Number: W002056805 (Inventor Roy et al), Minimally invasive glaucoma surgical instrument and method
Patent Number: W002074052 (Inventor Smedley et al), Applicator and methods for placing a trabecular shunt for glaucoma treatment
Patent Number WO03045290 (Inventor Conston et al), Ophthalmic Microsurgical System
The document WO 03043549 discloses a device comprising a probe with a suction channel for creating opening between anterior chamber und Schlemm's Canal, WO 03045290 and WO 03043549 are part of the prior art under Article 54(3) EPC.

### Brief Description of the Drawings

Figure 1 illustrates a sheath microcannula with an inner member.
Figure 2 illustrates a microcannula with expandable segments.
Figure 3 illustrates a microcannula with a signaling beacon tip.
Figure 4 illustrates a microcannula with a side connection fitting.
Figure 5 illustrates a microcannula with an open distal tip with a side channel for application of suction.
Figure 6 illustrates a microcannula with fenestrations and an inner member for controlled tissue removal.
Figure 7 illustrates a microcannula with a single fenestration for controlled tissue removal.
Figure 8 illustrates a microcannula with a rotating inner member for tissue cutting.
Figure 9 illustrates a microcannula with a side fenestration and tissue cutting flap.
Figure 10 illustrates a microcannula with a side fenestration and inner member for directed tissue abrasion.

### Description of Invention:

Schlemm's Canal is a channel in the corneo-scleral junction of the eye and is the primary pathway for the drainage of aqueous humor. The inner wall of the Canal comprises the TM and juxtacanalicular tissues through which the aqueous humor drains from the anterior chamber. The outer wall of is comprised of scleral tissue with openings to collector channels for the passage of aqueous humor from the Canal to the venous system. Due to its relative positioning to the TM, the Canal forms a circular channel that encircles the anterior chamber. The Canal is approximately 10 to 15 mm in diameter and 200 microns by 50 microns in cross-section. The drainage of aqueous humor through the TM and juxtacanalicular tissues into Schlemm's Canal is believed to be the predominant route for aqueous drainage. In open surgery for glaucoma, surgical treatment of the inner wall of Schlemm's Canal and removal of associated tissue such as the TM and juxtacanalicular tissues has demonstrated an increase in aqueous outflow and reduction of intraocular pressure. It is an object of the present invention to enable treatment and removal of tissues in these specific regions by use of minimally invasive surgical instruments. It is also an object of the invention to treat a specific segment of the tissue tract and also to treat specific regions of the selected segment to minimize surgical trauma and post-surgical scarring.

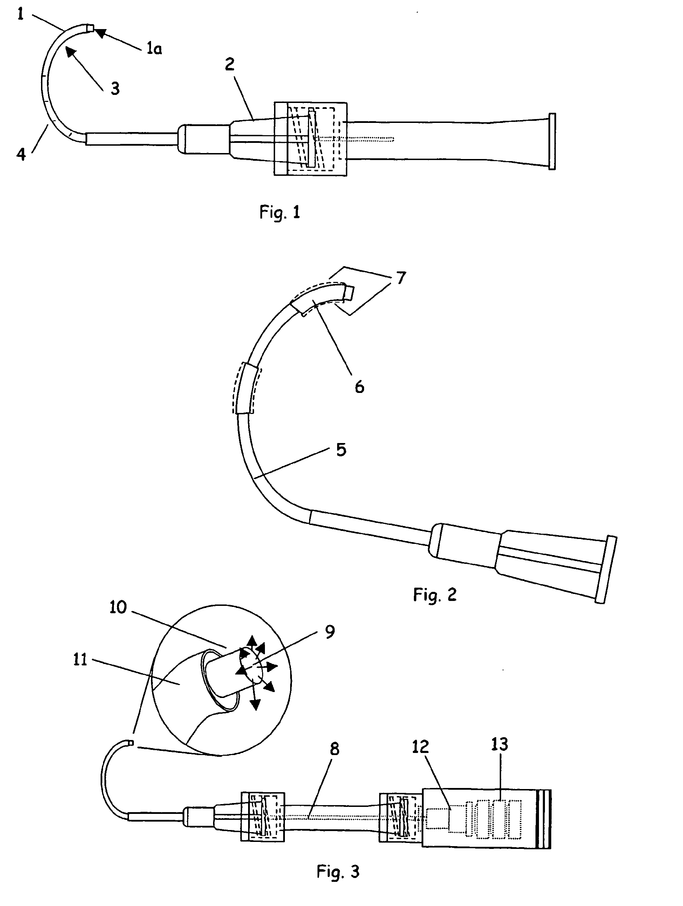

The ophthalmic microsurgical instruments of the present invention are defined by the features of claim 1 and dependent claims. They comprise a thin walled outer sheath microcannula with a connector at the proximal end, a distal tip and a communicating channel therebetween, as shown in Figure 1. The microcannula lumen provides a fluid and gas tight, sealed passage from the proximal end to the distal tip of the instruments. An inner member which fits and slides or rotates within the sheath may also be incorporated, the inner member comprising at least a proximal end and a distal tip. The distal end of the instruments may be curved in a manner to approximate the curvature of Schlemm's Canal. The instruments may also comprise a guidance means to effect proper advancement of the distal portion. Furthermore the instruments may comprise means to mechanically stabilize the target tissues. The tissues may be held in tension or compression for controlled treatment or removal of tissue. The instruments may also comprise cutting means to excise targeted tissues. The instruments may also be used to deliver drugs or implants to the tissue tract to treat adjacent tissues.

The microcannula may be introduced into Schlemm's Canal manually or as part of a system to provide surgical support or guidance. Once inserted into Schlemm's Canal, the microcannula may be progressively advanced to the appropriate areas for treatment. The distal end is preferably sized and curved or compliant enough to access at least one half the length of Schlemm's Canal, approximately 15 to 25 mm. Treatment of the entire Canal may be effected by inserting the instrument in the opposite direction from the first treatment at the surgical access point. The positioning of the instrument in the Canal can be verified by several means including a fiber-optic beacon tip inner member, a change in pressure or vacuum resistance in the surrounding environment as the system enters the Canal, a change in tissue color, direct visual location during surgical cut-down or by external image guidance such as ultrasound or optical coherence tomography. Features of the instrument can aid accurate positioning within the Canal.

The selective treatment or removal of TM and juxtacanalicular tissues adjacent to Schlemm's Canal may be accomplished by various means. One means incorporates the use of side holes or fenestrations on the outer sheath directed at the target tissues adjacent to the inner radius. The outer sheath may be configured to allow for tissue treatment or removal separately or in conjunction with an inner. member that works in alignment with the side holes or fenestrations. According to the invention selective treatment of the TM and juxtacanalicular tissues are accomplished by the use of suction through the microcannula, which has been observed to act predominantly on the inner wall of the Canal. Both means may be combined, such as the use of suction to pull a region of the target tissue into a side hole or fenestration of the outer sheath for subsequent treatment or excision.

Suction or vacuum may also be incorporated to clear the operative field and the microcannula lumen, either concurrent with tissue treatment or subsequent to tissue treatment since the sheath also functions to provide a disposal path for the excised tissues and surgical debris. Furthermore the ability of the cannula to remove particles and debris may be used by itself or in conjunction with other treatment methods such as laser trabeculoplasty in order to enhance the outcome by removal of waste particles.

The microcannula may comprise a thin walled polymer or metallic tube **1** of sufficient stiffness to allow it to be advanced into Schlemm's Canal, and of sufficient flexibility or compliance to follow the curvature of the Canal. It is preferable that the distal tip **1a** be beveled or radiused so as to provide for atraumatic advancement into the Canal. The proximal connector **2** may be of a Luer type or similar system for the attachment or introduction of secondary elements or may be designed for attachment only to specific components. Due to the small size of Schlemm's Canal, approximately 200 microns in diameter, the microcannula must be appropriately sized. Typically, the microcannula is sized in the range of 100 to 350 microns outer diameter with a wall thickness from 10 to 100 microns to allow cannulation of Schlemm's Canal. However, Schlemm's Canal may be expanded prior to insertion of the microcannula with for example, the injection of a surgical viscoelastic material. With prior expansion of the Canal, cannulation becomes much easier to perform without damaging tissues. Expansion of Schlemm's Canal also allows a microcannula of up to 500 microns outer diameter to be used to access the Canal.

Due to the curvature of Schlemm's Canal, the microcannula should be flexible in the appropriate dimensions. In some embodiments, a predetermined curvature **3** may be applied to the inner member and/or the outer sheath during fabrication. The curvature is preferably slightly greater than the curvature of the Canal in order to prevent the instrument from perforating the inner wall while advancing the microcannula. It is also desirable for a portion of the instrument to be able to be swiveled at least 180° around to provide for handedness to the curved microcannula. This allows the surgeon to cannulate the entire circumference of Schlemm's Canal from a comfortable working position.

Suitable materials for the microcannula sheath include metals, polyetheretherketone (PEEK), polyimide, polyamide, polysulfone, or similar materials. The sheath may also comprise surface treatments such as lubricious coatings to assist in cannulation and ultrasound or light interactive coatings to aid in location and guidance. The microcannula may also have markings **4** on the exterior for assessment of depth in the tissue tract. The external markings allow user assessment of the length of the tissue tract accessed by the microcannula, and the approximate location of the microcannula tip.

The microcannula **5** may also comprise a segment or series of segments capable of being expanded in a radial direction in order to place tension on the target tissues for treatment, as shown in Figure 2. The segments may comprise means such as stent-like structures, balloons or elastomeric sections **6** which may be inflated or deformed in a radial manner **7**. Multiple expandable segments may be used to stabilize and isolate segments of Schlemm's Canal for surgical or drug treatment through the microcannula lumen. Furthermore, the expandable segments may be slidably disposed about the central axis such that the segments may be translated axially apart from each other to provide further tension on the tissues. The expandable segments may comprise polymers and elastomers such as latex, silicone rubber, urethane, vinyl, polyether block amide (Pebax) or may be a metallic structure comprised of shape-memory or superelastic alloy, stainless steel, tungsten or similar materials. Alternatively, another outer member may be disposed about the microcannula as a tissue stabilization means. The expandable structure would be activated or mechanically released to expand during the procedure and then retracted or compressed for removal.

Depending on the application, the inner member may be used to guide the positioning of the microcannula, surgical tools and instrumentation or act as a surgical tool. The inner member may comprise a guide wire, hollow needle or tube, micro-trocar, cutting tool or similar element and comprises a proximal end and a distal tip, and may contain a communicating channel between. The inner member may also comprise sensing means such as a pressure transducer or fiber optic to aid in determining location, local fluid pressure, blood flow or other parameters. The inner element is sized correspondingly to fit slidably within the microcannula and therefore will be in the range of 90 to 450 microns in outer diameter. If hollow, the inner diameter will be in the range of 40 to 400 microns. The inner member may be removed during the surgical procedure and replaced sequentially with other inner members acting as instruments or tools.

A first inner member used for initial placement may comprise a signaling beacon to identify the location of the microcannula tip relative to the target tissues, as shown in Figure 3. The beacon may comprise an echogenic material for ultrasound guidance, an optically active material for optical guidance or a light source for visual guidance. In one embodiment, a plastic optical fiber (POF) **8** is used to provide a bright visual light source at its distal tip **9**. The distal tip of the POF **10** may be positioned at or slightly beyond the end of the microcannula sheath **11** and the emitted signal may be detected through the scleral tissues visually or using sensing means such as infrared imaging. The POF may also comprise a tip which is beveled or mirrored or otherwise configured to provide for a directional beacon. If the emitted directional light is directed toward the inner radius at the TM, the surgeon may view the illuminated spot in the anterior angle using a goniometer lens, and verify placement of the operative instrument at the targeted tissues. The beacon may be illuminated by a high intensity light source, laser, laser diode or light-emitting diode **12,** which may be powered by batteries **13** or standard AC power. Upon arrival of the microcannula distal end at the target tissues, the beacon assembly and POF may be removed, leaving the microcannula sheath at the desired location for treatment. The connection point between the outer microcannula sheath and the inner member may be sealed with a cap or preferably with a self-sealing mechanism such as a one-way valve or an elastomer seal.

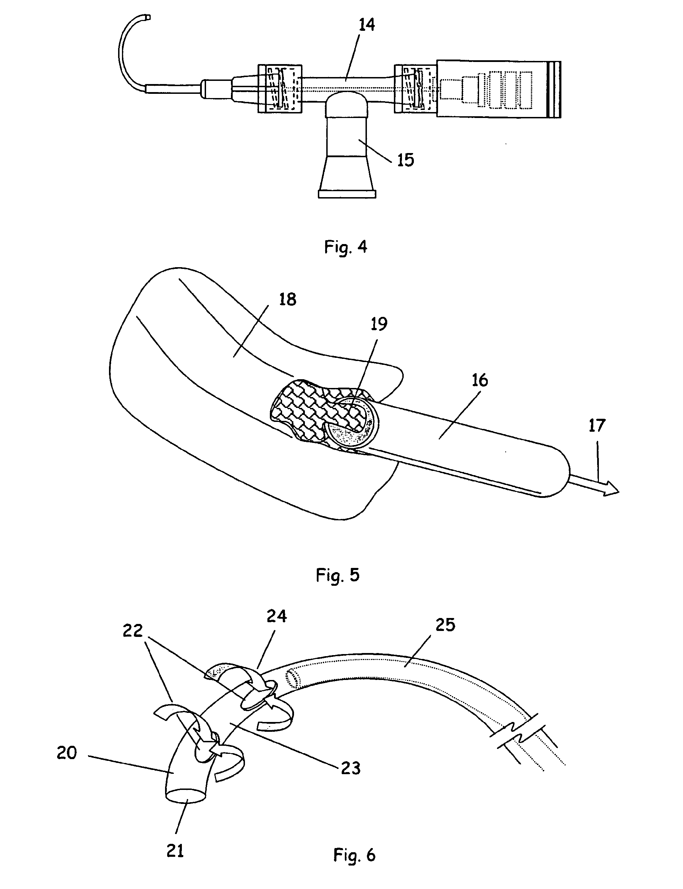

In one embodiment, the instrument set also comprises a fitting as the connection point for the illumination package. Additionally, as shown in Figure 4, the instrument may contain a central section **14** comprising a single or multiple side fittings **15** to allow the attachment of ancillary equipment such as syringes, vacuum or pressures sources, sensing means and the like. The attachment fittings may comprise standard designs such as Luer fittings or may be designed to only accept connection with specific components.

The operative function of the invention is an instrument to treat or remove specific tissues adjacent to Schlemm's Canal such as the TM in such a manner that the area of the treatment or removal is controlled and repeatable. In some applications, the instrument may be used to remove a controlled layer of adjacent target tissue, such as the juxtacanalicular tissues at the inner wall of Schlemm's Canal. Furthermore, the procedure can be performed at multiple sites within the eye to effect treatment per the patient's requirements by using the microcannula sheath for repositioning to other target locations from within the Canal.

In one embodiment the microcannula **16** alone is used to remove portions of the adjacent tissue using suction means **17,** as shown in Figure 5. The microcannula is advanced into Schlemm's Canal **18.** A vacuum syringe, vacuum or aspiration pump is used to provide suction and a portion of the inner wall is pulled into the lumen **19** and removed. Due the large difference in mechanical properties between the thick scleral outer wall of the Canal and the flexible tissues of the inner wall, suction applied by a microcannula has demonstrated preferential ability to manipulate the inner wall. Control of the suction characteristics may be used to control the amount of tissue treated or removed from the inner wall. In some cases, suction alone may be applied to the TM to remove tissue debris and improve aqueous outflow, without removing a portion of the TM.

In another embodiment, shown in Figure 6, the distal tip of the microcannula **20** is closed off **21.** A fenestration or series of fenestrations **22** are disposed along the inner radius wall **23** of the microcannula, directed toward the TM. Suction is applied **24** to pull a small amount of TM into the lumen and apply tension to the target tissue. An inner member **25,** comprised of a thin hollow shaft, is then extended through the microcannula **20** and may be rotated or axially advanced, to cut off the intruding tissues. The inner member may comprise a beveled or sharpened leading edge to facilitate tissue cutting. The excised tissue may be removed by a suction mechanism through the lumen. The amount of tissue removal may be controlled through the sizing of the ingress holes and the amount of suction applied. The outermost layer of the TM, including the juxtacanalicular tissues, interfacing Schlemm's Canal may be removed by minimal application of suction, or alternatively openings through the TM of controlled geometry may be formed with greater amounts of suction.

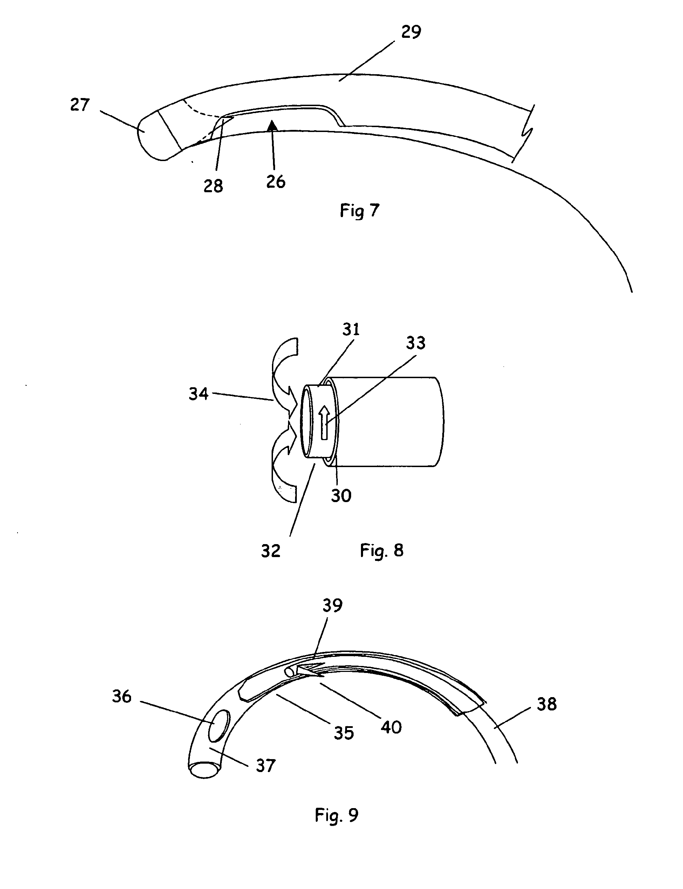

In a similar embodiment, Fig 7, a single fenestration **26** is created along the inner radius wall of the microcannula **29.** The distal tip **27** is closed and is fully radiused to produce a ball-end tip. A single cutting element **28** is disposed in the lumen at the distal end, with the cutting edge oriented proximally. The target tissues are pulled into the lumen, and the cannula is withdrawn which allows the cutting element to remove tissue to a determined depth. The cutting depth may be set and adjusted by the cutting element design, the dimensions of the fenestration and the amount of suction applied.

Furthermore, the microcannula may contain stabilization means in conjunction with cutting means thereby applying traction to the tissues to improve cutting efficiency and control. The microcannula may comprise a multilumen tube such that each lumen is connected separately to a hole or a series of holes along the inside radius facing the target tissues. For example, a two-lumen microcannula may be constructed comprised with three holes a set distance apart along the inner radius wall. The outermost two holes are connected to one lumen of the microcannula and the central hole to the second lumen. In this manner, a low suction pressure may be applied to the outermost holes, providing tissue stabilizing forces, while a higher suction pressure may be applied to the center hole, removing a controlled portion of tissue.

In another embodiment shown in Figure 8, the microcannula lumen is open **30** and a rotating hollow inner member **31** is employed. The distal tip of the inner member may be beveled or sharpened and is extended just slightly beyond the end of the cannula **32** or adjacent to a fenestration along the inner radius. Suction is applied to the cannula and the inner member is rotated **33** to provide a cutting action. As the instrument is advanced, the TM tissues are preferentially pulled **34** toward the microcannula axis allowing the inner member to cut away portions as required. The amount of tissue removal is controlled by extent of advancement and applied suction during the cutting process.

In another embodiment shown in Figure 9, the instrument distal end is comprised of two concentric thin-walled tubes. The outer tube **35** contains a window or fenestration **36** near the distal end and aligned along the inner radius wall of the tube **37** which interfaces the adjacent TM. The inner tube **38** contains an angled slit **39** partially through the tube which creates a sharp pointed flap **40** directed proximally and also aligned with the window in the outer tube and the TM. The flap **40** is prebent to allow it to project outward from the tubing **38,** in the direction of the TM and is used as a piercing and cutting member. The outer tube **35** is slidably disposed about the inner tube. During insertion into Schlemm's Canal, the outer tube is positioned such that the window is not adjacent to the flap and the flap is thereby constrained within the outer tube. At the operative target position, the outer tube is advanced so that the window is over the flap, allowing the flap to protrude from the assembly. The instrument is retracted slightly allowing the flap to pierce the TM and then retracted a specified amount such that the full length of the flap has pierced the tissues. The outer tube is then retracted, moving the window proximally, causing the flap to be pulled back and thereby cutting a portion of the TM approximating the geometry of the flap and constraining the excised tissue within the inner tube for disposal.
Suction may be used to remove the tissue from the lumen and the procedure repeated as required.

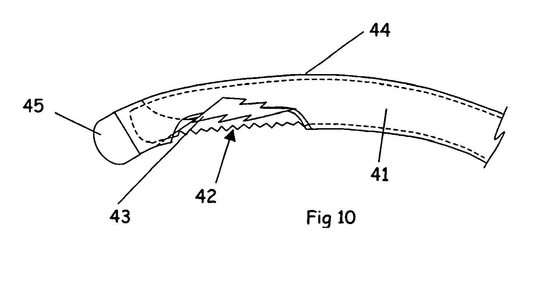

In another embodiment shown in Figure 10, the inner wall of Schlemm's Canal may be removed by the application of controlled abrasion. The inner member **41** may comprise a brush or rasp like tool **42** on the distal end which abrades the tissue surface. The abrading tool may be used by passing the distal portion of the inner member past the distal tip of the microcannula with concurrent suction, or by positioning it in a window or fenestration **43** in the side of the microcannula **44** near the distal tip **45.** The use of a side opening allows a controlled portion of the tissue tract, such as the TM adjacent to Schlemm's Canal to be treated selectively. Suction may also be applied concurrently through the microcannula lumen to stabilize the tissues during treatment and remove resultant tissue debris.

The microcannula may also be used to deliver a fiber optic for laser ablation of the tissues from within Schlemm's Canal. The microcannula may be used to provide suction to remove the ablative residue and any tissue debris from the site and deliver treatment adjuvants or medications to minimize fibrosis during wound healing.

### Examples:

Example 1: A single element microcannula was fabricated with polyimide tubing (MicroLumen, Inc.), 0.0101" (256µ) inner diameter by 0.0141" (358µ) outer diameter. The distal end was sealed with epoxy to create a ball end. The distal portion was curved with a radius of approximately 15mm for a distance of 2 cm. A fenestration approximately 1.2 mm long was cut into the inner wall of the curvature and extending inward to 1/2 the diameter. A Luer fitting was bonded to the proximal end. The microcannula was attached to a collection bottle and then to a vacuum pump generating up to 685.8mm [27 inches] of Hg.

An enucleated human eye was prepared for the experiment by inflating the posterior chamber to a pressure of 10mm Hg with phosphate buffered saline (PBS). A scleral flap was surgically excised and Schlemm's Canal unroofed. The microcannula was inserted into Schlemm's Canal and vacuum was applied. Suction was confirmed by observing fluid flow within the microcannula.

Subsequently, the globe was hemisected and the vitreous, ciliary body, lens and iris removed allowing visualization of the TM and Schlemm's Canal from inside. The microcannula was advanced into the Canal to a point approximately 100° from the surgical site. Suction was applied and the results observed visually under the surgical microscope. Upon application of vacuum, the inner wall of Schlemm's Canal at the fenestration site was seen to be pulled into the lumen of the microcannula.
The vacuum level was varied from 25.4 to 685.8mm [1 to 27 inches] Hg. In each case the inner wall was observed being pulled into the lumen at approximately 101.6mm [4 inches] Hg or greater, while the outer wall was not noticeably deformed. The microcannula was withdrawn under vacuum and upon examination, excised tissue was observed adhered to the distal edge of the fenestration. An open ended microcannula of approximately the same size, without side fenestration, was placed in Schlemm's Canal and the suction experiments repeated at various vacuum levels. The inner wall of the Canal was observed to be preferentially deflected toward the microcannula tip at approximately 101.6mm [4 inches] of Hg or greater.

Example 2: A microcannula with an inner member and outer sheath was fabricated. The outer sheath was fabricated with a single fenestration as in Example 1 but with a polyimide tube of 221 microns [0.0087"] inner diameter and 297 microns [0.0117"] outer diameter. The inner member was comprised of polyimide tubing 124 microns [0.0049"] inner diameter by 170 microns [0.0067"] outer diameter and was slidably disposed within the outer member.

An enucleated human eye was prepared as in Example 1. The microcannula was placed with the fenestration toward the inner wall of Schlemm's Canal. The vacuum was applied and tissue was seen being pulled into the lumen. The inner member was then advanced until it stopped against the closed distal tip of the outer member. Upon removal of the microcannula, excised tissue was observed attached to the inner member.

Example 3: A microcannula with an inner member and outer sheath was fabricated. The outer sheath was similar to the outer sheath in Example 2. An inner member designed to abrade the tissues was fabricated comprised of a stainless steel wire 152 microns [0.006"] diameter to which the distal end was roughened using a grinding wheel. The inner member was slidably disposed within the outer sheath.

An enucleated human eye was prepared as in Example 1 with the addition of placing a 27 gauge needle into the cornea, and attaching the needle to a flow meter and reservoir of PBS. The reservoir was raised to provide constant pressure flow into the anterior chamber, and the flow meter used to observe changes in flow.

The microcannula was advanced into Schlemm's Canal. Suction was applied to pull the inner wall of the Canal into the lumen and then the inner member was slid back and forth across the tissues. The microcannula was removed and surgical flap sealed. An increase in aqueous outflow was observed after the procedure.

Example 4: A microcannula was fabricated similar to the outer sheath in Example 2. A cutting element inner member was fabricated from Nitinol wire, incorporating a flat blade situated at the axis of the wire. The cutting element was bonded into the distal lumen of the microcannula with the cutting blade facing proximally and extending into the fenestration area.

Enucleated human eyes were prepared as in Example 3. The microcannula was advanced into Schlemm's Canal. Suction was applied, drawing the inner wall of the Canal into the lumen, and the microcannula was retracted while still under vacuum. Upon removal from the eye, the cutting element was observed to have excised tissue attached. Subsequently aqueous outflow was seen to increase.

Example 5: A signaling means for determining the location of the microcannula was fabricated and incorporated into a microcannula instrument. A single strand plastic optical fiber (POF) (Biogeneral, Inc.) 100 microns in diameter was used with a flat distal tip. The fiber was disposed within an instrument assembly comprising a polyimide microcannula 110 microns ID and 160 microns OD (MicroLumen, Inc.), which was bonded to a needle assembly. The needle assembly consisted of a base section of 18 gauge hypodermic tubing, with a 14 gauge tubing guide tube fabricated so as to slide forward and backward along the 18 gauge tube for a fixed distance of 15 mm. The distal tip of the guide tube was comprised of a 28 gauge tube to direct the microcannula and POF during insertion. The POF was illuminated using a battery powered red laser diode (Digikey Corp.). A second POF was also fabricated with a distal tip cut at approximately 60° and the jacket removed opposite the bevel. This provided a partially directed illumination spot toward the inner radius.

An ex-vivo human eye was placed in a soft holding cup stage under a stereomicroscope. A surgical flap was created at the limbus and the flap removed to access Schlemm's Canal. The tip of the guide tube was placed at the ostium of the Canal. The microcannula and POF were advanced into the canal with the light source on. The illuminated tip of the fiber was seen through the scleral tissues in the case of the flat tipped POF. Using the beveled POF, illumination could be viewed from within the anterior chamber of the eye depending on the rotation of the microcannula, allowing the appropriate surgical tissues such as the TM to be targeted.

Example 6: In another example, Schlemm's Canal of an eye is cannulated with the microcannula described in example 3. The signaling beacon inner member is used to verify the position of the tip of the microcannula in the desired location of the eye and with proper rotational alignment with respect to the TM. The signaling beacon inner member is removed and a surgical tool inner member to remove tissue from the TM is guided into the lumen of the microcannula and advanced to the distal tip. The inner member also incorporates suction to remove tissue debris. After removal of TM tissue, the surgical tool inner member is exchanged for the signal beacon inner member. The microcannula may be positioned to another area of Schlemm's Canal to repeat the process as needed to increase aqueous outflow to an appropriate level.

While the present invention has been described herein with respect to the exemplary embodiments and the best mode for practicing the invention, it will be apparent to one of ordinary skill in the art that many modifications, improvements and subcombinations of the various embodiments, adaptations and variations can be made to the invention without departing from the scope of the appended claims.

## Claims

1. A microcannula (5) based microsurgical device designed to operate within Schlemm's Canal of the eye and to treat a controlled amount of trabecular meshwork and juxtacanalicular tissues adjacent to the inner radius of Schlemm's Canal (18), the device comprising:
a flexible tubular sheath (1) having proximal (2) and distal (1 a) ends, and configured to fit within Schlemm's Canal (18);
a distal assembly for sealed introduction and removal of materials and tools;
**characterised in that** said sheath has an outer diameter of no more than 500 microns, and
wherein, in use, suction is provided through the microcannula sheath to position adjacent tissue to be removed into a lumen extending through the tubular sheath.

2. A microcannula based microsurgical device as described in claim 1, wherein the microcannula (20) has one or more openings (22) directed toward an inner radius thereof.

3. A microcannula based microsurgical device as described in claim 1 or claim 2,
wherein the suction level is at least 100mm [4 inches] of Hg.

4. A microcannula based microsurgical device as described in any of the preceding claims, further comprising at least one inflatable or expandable member to provide sealing of Schlemm's Canal (18) during treatment.

5. A microcannula based microsurgical device as described in any of the preceding claims, wherein the microcannula (32) additionally comprises an inner member (31) with a proximal end and a distal tip;
and wherein the sheath and inner member are sized such that the inner member fits slidably within the sheath and the distal tip of the inner member acts to treat adjacent tissue through one or more openings in the distal end of the microcannula,
and preferably wherein the inner member acts to remove tissues from an inner wall of Schlemm's Canal.

6. A microcannula based microsurgical device as claimed in claim 1 further comprising:
an inner member with a proximal end and a distal tip sized such that the inner member fits slidably within the sheath,
wherein the sheath has one or more openings directed toward an inner radius at the distal end,
and the sheath and inner member act to remove adjacent tissue through the one or more openings in the distal end of the sheath.

7. A microcannula based microsurgical device as in claim 6, wherein suction can be provided through the lumen during removal of adjacent tissue.

8. A microcannula based microsurgical device as described in claim 6 or claim7,
wherein the distal tip of the inner member is shaped for tissue dissection, cutting, ablation or removal.

9. A microcannula based microsurgical device as described in any of claims 6-8,
wherein the inner member performs removal of tissue within the lumen of the tubular sheath.

10. A microcannula based microsurgical device as described in any of the preceding claims, further comprising a plurality of markers (4) set at regular intervals along the tubular sheath such that each marker is spaced from adjacent markers by a fixed distance along the sheath to provide depth measurement.

11. A microcannula based microsurgical device as described in any of the preceding claims, wherein, the tubular sheath additionally comprises materials to enhance observation of the device positioning under image guidance.

12. A microcannula based microsurgical device as described in any of the preceding claims, wherein the tubular sheath comprises a polyimide or a fluoropolymer.

13. A microcannula based microsurgical device as described in any of the preceding claims, wherein the microcannula has a length of at least 15 mm.

14. A microcannula based microsurgical device as described in any of the preceding claims, wherein the flexible tubular sheath is curved in the range of 10-15 mm diameter.

15. A microcannula based microsurgical device as described in any of claims 6-14,
wherein the inner member is curved in the range of 10-15 mm diameter.

16. A microcannula based microsurgical device as described in any of claims 6-15,
wherein the outer member is formed of a multi-lumen tube.

17. A microcannula based microsurgical device as described in any of claims 6-16,
wherein the inner member comprises steel, nickel titanium alloy or tungsten.

18. A microcannula based microsurgical device as described in any of claims 6-16,
wherein the inner member comprises an optical fiber, preferably wherein illumination from the optical fiber is directed from the distal end of the microcannula at an angle of 45 to 135 degrees from an axis of the microcannula to be coincident with an area of tissue removal.

19. A microcannula based microsurgical device as described in any of the preceding claims further comprising at least one inflatable or expandable member to provide stabilization of the device and surrounding tissues.

## Patentansprüche

1. Mikrochirurgisches Gerät auf Basis einer Mikrokanüle (5), das zur Betätigung innerhalb des Schlemmschen Kanals des Auges und zur Behandlung einer kontrollierten Menge des Trabekelwerks und von juxtakanalikulären Gewebe benachbart dem Innenrand des Schlemmschen Kanals (18) gestaltet ist,
wobei das Gerät aufweist:
Eine flexible röhrenförmige Hülse (1) mit einem proximalen (2) und einem distalen (1a) Ende, die dazu gestaltet ist, um in den Schlemmschen Kanal (18) zu passen,
einen distalen Aufbau zur abgedichteten Einführung und Entfernung von Materialien und Werkzeugen,
**dadurch gekennzeichnet, dass** die Hülse einen Außendurchmesser von nicht mehr als 500 µm hat, und
wobei im Betrieb ein Ansaugen durch die Hülse der Mikrokanüle bereitgestellt wird, um benachbartes, zu entfernendes Gewebe in ein durch die röhrenförmige Hülse verlaufendes Lumen zu bringen.

2. Mikrochirurgisches Gerät auf Basis einer Mikrokanüle nach Anspruch 1, wobei die Mikrokanüle (20) eine oder mehrere Öffnungen (22) hat, die auf einen inneren Radius davon gerichtet sind.

3. Mikrochirurgisches Gerät auf Basis einer Mikrokanüle nach Anspruch 1 oder 2, wobei der Ansaugdruck wenigstens 100 ml (4 Zoll) Hg beträgt.

4. Mikrochirurgisches Gerät auf Basis einer Mikrokanüle nach einem der vorhergehenden Ansprüche, das weiter wenigstens ein aufblasbares oder expandierbares Teil hat, um während der Behandlung eine Abdichtung des Schlemmschen Kanals (18) zu erreichen.

5. Mikrochirurgisches Gerät auf Basis einer Mikrokanüle nach einem der vorhergehenden Ansprüche, wobei die Mikrokanüle (32) weiter eine innere Komponente (31) mit einem proximalem Ende und einer distalen Endspitze aufweist,
und wobei die Hülse und die innere Komponente derartige Abmessungen haben, dass die innere Komponente gleitfähig in die Hülse passt und die distale Endspitze der inneren Komponente zur Behandlung auf benachbartes Gewebe durch eine oder mehrere Öffnungen in dem distalen Ende der Mikrokanüle wirkt,
und wobei vorzugsweise die innere Komponente dazu dient, um Gewebe von einer inneren Wand des Schlemmschen Kanals zu entfernen.

6. Mikrochirurgisches Gerät auf Basis einer Mikrokanüle nach Anspruch 1, das ferner aufweist:
Eine innere Komponente mit einem proximalen Ende und einer distalen Endspitze mit solchen Abmessungen, dass die innere Komponente gleitfähig in die Hülse passt,
wobei die Hülse am distalen Ende eine oder mehrere Öffnungen gerichtet auf einen Innenradius hat,
und die Hülse und die innere Komponente dazu wirken, um benachbartes Gewebe durch die eine oder die mehreren Öffnungen in dem distalen Ende der Hülse zu entfernen.

7. Mikrochirurgisches Gerät auf Basis einer Mikrokanüle nach Anspruch 6, wobei während der Entfernung des benachbarten Gewebes eine Saugwirkung durch das Lumen bereitgestellt werden kann.

8. Mikrochirurgisches Gerät auf Basis einer Mikrokanüle nach Ansprüchen 6 oder 7, wobei die distale Spitze der Innenkomponente so geformt ist, um Gewebe zu sezieren, schneiden, abzutragen oder zu entfernen.

9. Mikrochirurgisches Gerät auf Basis einer Mikrokanüle nach einem der Ansprüche 6 - 8, wobei die innere Komponente die Entfernung vom Gewebe innerhalb des Lumens der röhrenförmigen Hülse bewirkt.

10. Mikrochirurgisches Gerät auf Basis einer Mikrokanüle nach einem der vorhergehenden Ansprüche, das weiter eine Mehrzahl von Markierungen (4) in regelmäßigen Abständen entlang der röhrenförmigen Hülse aufweist, so dass jede Markierung in einem fixierten Abstand zu den benachbarten Markierungen entlang der Hülse liegt, um eine Tiefenmessung zu ermöglichen.

11. Mikrochirurgisches Gerät auf Basis einer Mikrokanüle nach einem der vorhergehenden Ansprüche, wobei die röhrenförmige Hülse Materialien aufweist, um die Beobachtbarkeit der Geräteposition unter Bildführung zu verbessern.

12. Mikrochirurgisches Gerät auf Basis einer Mikrokanüle nach einem der vorhergehenden Ansprüche, wobei die röhrenförmige Hülse ein Polyimid oder ein Fluorpolymer aufweist.

13. Mirkochirurgisches Gerät auf Basis einer Mikrokanüle nach einem der vorhergehenden Ansprüche, wobei die Mikrokanüle eine Länge von wenigstens 15 mm hat.

14. Mikrochirurgisches Gerät auf Basis einer Mikrokanüle nach einem der vorhergehenden Ansprüche, wobei die flexible röhrenförmige Hülse gekrümmt ist mit einem Durchmesserbereich von 10 bis 15 mm.

15. Mikrochirurgisches Gerät auf Basis einer Mikrokanüle nach einem der Ansprüche 6 - 14 wobei die innere Komponente mit einem Durchmesserbereich von 10 bis 15 mm gekrümmt ist.

16. Mikrochirurgisches Gerät auf Basis einer Mikrokanüle nach einem der Ansprüche 6 - 15, wobei das äußere Teil als mehrlumige Röhre gebildet ist.

17. Mikrochirurgisches Gerät auf Basis einer Mikrokanüle nach einem der Ansprüche 6 - 16, wobei die innere Komponente Stahl, Nickeltitanlegierung oder Wolfram aufweist.

18. Mikrochirurgisches Gerät auf Basis einer Mikrokanüle nach einem der Ansprüche 6 - 16, wobei die innere Komponente eine optische Faser aufweist, wobei vorzugsweise Beleuchtung aus der optischen Faser von dem distalen Ende der Mikrokanüle unter einem Winkel von 45 bis 135 Grad von der Achse der Mikrokanüle gerichtet wird, um mit einem Gebiet der Gewebeentfernung übereinzustimmen.

19. Mikrochirurgisches Gerät auf Basis einer Mikrokanüle nach einem der vorhergehenden Ansprüche, das weiter wenigstens ein aufblasbares oder expandierbares Teil aufweist, um eine Stabilisierung des Geräts und der umgebenden Gewebe zu erreichen.

## Revendications

1. Dispositif microchirurgical à base d'une microcanule (5) mis au point pour fonctionner à l'intérieur du canal de Schlemm de l'oeil et pour traiter une quantité contrôlée de tissus de trabéculum cornéoscléral et juxta-canaliculaires adjacents au rayon interne du canal de Schlemm (18), le dispositif comprenant :
- une gaine tubulaire flexible (1) ayant des extrémités proximale (2) et distale (1a), et configurée pour s'adapter à l'intérieur du canal de Schlemm (18) ;
- un ensemble distal pour une introduction et un retrait scellés de manière étanche de matières et d'outils ;
- **caractérisé par le fait que** ladite gaine a un diamètre externe de pas plus de 500 microns, et
- dans lequel, lors de l'utilisation, une aspiration est produite à travers la gaine de microcanule pour positionner un tissu adjacent devant être retiré à l'intérieur d'une lumière s'étendant à travers la gaine tubulaire.

2. Dispositif microchirurgical à base d'une microcanule selon la revendication 1, dans lequel la microcanule (20) a une ou plusieurs ouvertures (22) dirigées vers un rayon interne de celle-ci.

3. Dispositif microchirurgical à base d'une microcanule selon la revendication 1 ou la revendication 2, dans lequel le niveau d'aspiration est d'au moins 100 mm [4 pouces] de Hg.

4. Dispositif microchirurgical à base d'une microcanule selon l'une quelconque des revendications précédentes, comprenant en outre au moins un élément gonflable ou expansible pour fournir un scellement étanche du canal de Schlemm (18) pendant un traitement.

5. Dispositif microchirurgical à base d'une microcanule selon l'une quelconque des revendications précédentes, dans lequel la microcanule (32) comprend en outre un élément interne (31) avec une extrémité proximale et une pointe distale ; et dans lequel la gaine et l'élément interne sont dimensionnés de telle sorte que l'élément s'ajuste de façon coulissante à l'intérieur de la gaine et la pointe distale de l'élément interne agit pour traiter un tissu adjacent à travers une ou plusieurs ouvertures dans l'extrémité distale de la microcanule,
et, de préférence, dans lequel l'élément interne agit pour retirer des tissus à partir d'une paroi interne du canal de Schlemm.

6. Dispositif microchirurgical à base d'une microcanule selon la revendication 1, comprenant en outre :
- un élément interne avec une extrémité proximale et une pointe distale, dimensionné de telle sorte que l'élément interne s'ajuste de façon coulissante à l'intérieur de la gaine,
- dans lequel la gaine a une ou plusieurs ouvertures dirigées vers un rayon interne à l'extrémité distale, et
- la gaine et l'élément interne agissent pour retirer un tissu adjacent à travers la ou les ouvertures dans l'extrémité distale de la gaine.

7. Dispositif microchirurgical à base d'une microcanule selon la revendication 6, dans lequel l'aspiration peut être produite à travers la lumière pendant un retrait de tissu adjacent.

8. Dispositif microchirurgical à base d'une microcanule selon la revendication 6 ou la revendication 7, dans lequel la pointe distale de l'élément interne est façonnée pour une dissection, une découpe, une ablation ou un retrait de tissu.

9. Dispositif microchirurgical à base d'une microcanule selon l'une quelconque des revendications 6-8, dans lequel l'élément interne effectue un retrait de tissu à l'intérieur de la lumière de la gaine tubulaire.

10. Dispositif microchirurgical à base d'une microcanule selon l'une quelconque des revendications précédentes, comprenant en outre une pluralité de marqueurs (4) disposés à des intervalles réguliers le long de la gaine tubulaire, de telle sorte que chaque marqueur est espacé des marqueurs adjacents d'une distance fixe le long de la gaine pour fournir une mesure de profondeur.

11. Dispositif microchirurgical à base d'une microcanule selon l'une quelconque des revendications précédentes, dans lequel la gaine tubulaire comprend en outre des matières pour améliorer l'observation du positionnement du dispositif sous guidage par image.

12. Dispositif microchirurgical à base d'une microcanule selon l'une quelconque des revendications précédentes, dans lequel la gaine tubulaire comprend un polyimide ou un polymère fluoré.

13. Dispositif microchirurgical à base d'une microcanule selon l'une quelconque des revendications précédentes, dans lequel la microcanule a une longueur d'au moins 15 mm.

14. Dispositif microchirurgical à base d'une microcanule selon l'une quelconque des revendications précédentes, dans lequel la gaine tubulaire flexible est incurvée dans la plage de 10-15 mm de diamètre.

15. Dispositif microchirurgical à base d'une microcanule selon l'une quelconque des revendications 6-14, dans lequel l'élément interne est incurvé dans la plage de 10-15 mm de diamètre.

16. Dispositif microchirurgical à base d'une microcanule selon l'une quelconque des revendications 6-15, dans lequel l'élément externe est formé d'un tube à multiples lumières.

17. Dispositif microchirurgical à base d'une microcanule selon l'une quelconque des revendications 6-16, dans lequel l'élément interne comprend de l'acier, un alliage de nickel et de titane ou du tungstène.

18. Dispositif microchirurgical à base d'une microcanule selon l'une quelconque des revendications 6-16, dans lequel l'élément interne comprend une fibre optique, de préférence dans lequel l'éclairement provenant de la fibre optique est dirigé à partir de l'extrémité distale de la microcanule à un angle de 45 à 135 degrés par rapport à un axe de la microcanule pour coïncider avec une zone de retrait de tissu.

19. Dispositif microchirurgical à base d'une microcanule selon l'une quelconque des revendications précédentes, comprenant en outre au moins un élément gonflable ou expansible pour fournir une stabilisation du dispositif et des tissus environnants.
